Europäisches Patentamt

European Patent Office

Office européen des brevets

(11) Publication number: **0 242 062**
**A2**

(12) **EUROPEAN PATENT APPLICATION**

(21) Application number: 87302371.7

(22) Date of filing: 19.03.87

(51) Int. Cl.⁴: **C12N 15/00** , C12Q 1/68 , A01H 1/02

The applicant has filed a statement in accordance with Rule 28 (4) EPC (issue of a sample only to an expert). Accession number(s) of the deposit(s): Nr. IVI - 10107.

(30) Priority: **20.03.86 US 841594**

(43) Date of publication of application:
**21.10.87 Bulletin 87/43**

(84) Designated Contracting States:
**AT BE CH DE ES FR GB GR IT LI LU NL SE**

(71) Applicant: **LUBRIZOL GENETICS INC.**
**3375 Mitchell Lane**
**Boulder Colorado 80301-2244(US)**

Applicant: **The Regents of New Mexico State University**
**Box 3699**
**Las Cruces New Mexico 88003(US)**

(72) Inventor: **Tanksley, Steven D.**
**404 Winston Court No. 1**
**Ithaca New York 14853(US)**
Inventor: **Bernatzky, Robert**
**519 East Buffalo No. 1**
**Ithaca New York 14853(US)**

(74) Representative: **Fisher, Adrian John et al**
**CARPMAELS & RANSFORD 43 Bloomsbury Square**
**London WC1A 2RA(GB)**

(54) **Tomato chromosome map and cDNA library.**

(57) This invention provides a map of tomato chromosomes marked with the location of a large number of cDNA clones and isozyme markers. The cDNA clones are on deposit with In Vitro International, and may be used in conjunction with the map by finding linkages between desirable breeding characteristics of a tomato plant. After such linkages are established, they may be used to quickly determine the presence or absence of the desired characteristics in tomato plant and to introgress such desired characteristics into another tomato variety.

EP 0 242 062 A2

FIG. I

## TOMATO CHROMOSOME MAP AND cDNA LIBRARY

Field of the Invention

This invention lies in the field of genetic engineering using recombinant DNA clones, and in the field of plant breeding.

Background of the Invention

Genetic linkage maps have been developed for a wide variety of organisms and have been invaluable in the introgression of specific chromosomes or chromosome segments into various genetic backgrounds (Rick, C.M. and Khush, G.S. (1969) "Cytogenic explorations in the tomato genome", pp. 45-68, In: Genetics Lectures, Vol. I (R. Bogart ed.), Oregon State University Press, Corvalis; and C. Rhyne (1960) "Linkage studies in Gossypium II altered recombination values in linkage group of allotetraploid G. hirsutum L. as a result of transferred diploid species genes. Genetics 45:673-683). Localization of genes of interest can also be accomplished through linkage analysis with mapped markers as described by Patterson, E.B. (1982) "The mapping of genes by the use of chromosomal aberrations and multiple marker stocks", pp. 85-88, In: Maize for Biological Research (W.F. Sheridan, ed.) University Press, University of North Dakota, incorporated herein by reference.

Among higher plants, tomato ( Lycopersicon esculentum) has one of the most extensive linkage maps. Marker loci have been used in tomato for the construction of chromosome substitution lines (Rick, C.M. (1969) "Controlled introgression of chromosomes of Solanum pennellii into Lycopersicon esculentum: segregation and recombination", Genetics 62:753-768; and Rick, C.M. (1971) "Some cytogenic features of the genome in diploid plant species", Stadler Symposia Vol. 1/2:153-174) and as an aid in the identification of genes involved in self-incompatibility (Tanksley, S.D. and Loaiza-Figueroa, F. (1985) "Genetic self incompatibility is controlled by a single major locus on chromosome 1 in Lycopersicon peruvianum, Proc. Nat'l. Acad. Sci. 82:5093-5096). The introgression of specific genes of economic importance such as male sterility (Tanksley, S.D., Rick, C.M. and Vallejos, C.E. (1984), "Tight linkage between a nuclear male-sterile locus and an enzyme marker in tomato", Theor. Appl. Genet. 68:109-113) and nematode resistance (Medina-Filho, H.P. (1980), "Linkage of Aps-1, mi and other markers on chromosome 6", Rep. Tomato Genet. Coop. 30:26-28) has also been facilitated by the use of more easily selected linked marker loci.

Though the genetic map of tomato is well-populated with markers, it is, like most plant species, made up of primarily morphological mutant loci. In general, these morphological markers are not useful in selection programs for plant breeding since they are normally recessive and have undesirable phenotypes.

The development of electrophoresis and enzyme specific stains has expanded the map in tomato and has provided many new markers, the variants of which do not often have any noticeable effect on phenotype. These isozyme markers, though highly useful, are few in number and cover only a fraction of the tomato genome (Tanksley, S.D. and Rick, C.M. (1980), "Isozyme gene linkage map of the tomato: applications in genetics and breeding", Theor. Appl. Genet. 57:161-170; Tanksley, S.D. (1985), "Enzyme coding genes in tomato (Lycopersicon esculentum), Isozyme Bulletin 18 :43-45). For these reasons, it was desired to develop a more saturated linkage map in tomato based on and unique DNA sequences derived from messenger RNA (cDNA).

In recent years, unique DNA sequences corresponding to specific chromosomal sites have been used as genetic markers in humans (Botstein, D., White, R.L., Skolnick, M. and Davis, R. (1980), "Construction of a genetic linkage map in man using restriction fragment length polymorphisms", Am. J. Hum. Genet. 32:314-331; Jeffreys, A.J., Wilson, V. and Thein, S.L. (1985), "Hypervariable 'minisatellite' regions in human DNA", Nature 314:67-73; and White, R., Leppert, M., Bishop, D.T., Barker, D., Berkowitz, J., Brown, C., Callahan, P., Holm, T., Jerominski, L. (1985), "Construction of linkage maps with DNA markers for human chromosomes", Nature 313:101-105); and it has been suggested that this method be used in plants (Helentjaris, T.G., PCT Application published December 6, 1984, "Process for genetic mapping and cross-breeding thereon for plants"). With this approach, allelic variation is detected by first digesting DNA from the individuals being analyzed with a variety of restriction endonucleases. The resulting fragments are separated by electrophoresis and transferred to membranes. Allelic fragments are then identified by probing filters with radioactively-labelled, cloned, homologous sequences. Genetic variation detected in this manner has often been referred to as restriction fragment length polymorphism (RFLP). The number of RFLPs are virtually unlimited. They have no effect on phenotype, are codominant and are inherited in a Mendelian fashion (Botstein et al. 1980, supra).

Mapping of loci detected by RFLP's has advanced rapidly in humans and the information is finding application in prenatal diagnosis of a number of genetic disorders (Murray, J.M., Davies, K.E., Harper, P.S., Meredith, L., Mueller, C.R., Williamson, R. (1982), "Linkage relationships of a cloned DNA sequence on the short arm of the X chromosome to Duchenne muscular distrophy", Nature 300:69-70). While human geneticists have been rapidly developing maps using RFLP's (Solomon, F. and Goodfellow, P. (1983), "Human gene mapping rolls along", Nature 306:223-224), such mapping in higher plants, to this point, has been limited (Polans, N.O., Weeden, N.F. and Thompson, W.F. (1985), "Inheritance, organization and mapping of rbcs and cab multigene families in pea", Proc. Nat'l. Acad. Sci. 32:5083-5087). Specifically, mapped cloned DNA sequences have not been previously reported in connection with tomato plants.

DNA markers in tomato will provide a basis for early screening procedures for many simply inherited characteristics as well as insight into the genetic organization of complex traits. Such screening procedures may be used for the introgression of desirable traits into tomato varieties. A well-populated linkage map based on DNA sequences can also provide information about the evolution of plant genomes. Plant DNA fragments that hybridize to individual probes can be considered truly homologous and the genomic distribution of duplications and multi-gene family members can thus be compared among different genera.

Summary of the Invention

This invention provides a map of tomato chromosomes marked with the location of a large number of cDNA clones and isozyme markers. The cDNA clones are on deposit with In Vitro International, and may be used in conjunction with the map by finding linkages between the clones and desirable, or observable breeding characteristics of a tomato plant. These linkages are established by means known to the art by comparing the percent recombination of the desired characteristic with the cDNA marker clones and designating the cDNA marker clone having the lowest percent recombination, preferably less than 30%, and more preferably less than 10%, with the desired characteristic, as being linked with the desired characteristic. The desired characteristic may be mapped on the tomato genome by placing it a calculated number of map units from previously mapped cDNA markers based on its recombination rate with these markers. Linkages between cDNA markers and observable characteristics may be used to quickly determine the presence or absence of the characteristics in particular tomato plants by testing for the presence of RFLP's characteristic of the linked markers. This ability to test genetic material without the necessity for growing up and breeding all plants is useful in breeding programs to introgress desired characteristics from one tomato variety into another.

Description of the Figures

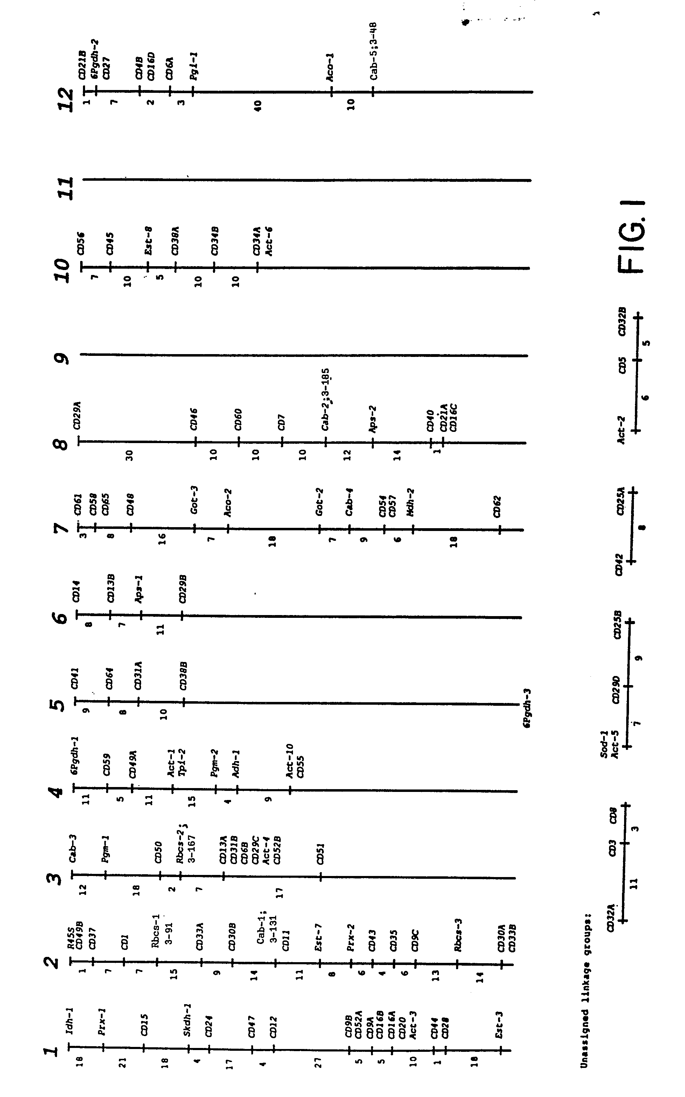

Figure 1 shows a tomato linkage map based on cDNA and isozyme markers. cDNA loci of this invention are designated by CD number or clone number. Alphabetical designations, A, B, C, D, following the CD number are used only to indicate first, second and multiple loci for the clone. The name of the clone with the alphabetical designation is considered equivalent to the name of the clone without the alphabetical designation. Other DNA markers are the large subunit of ribosomal RNA (R45s), the small subunit of ribulose bisphosphate carboxylase (Rbcs), the major chlorophyll a/b binding protein (Cab) and actin (Act). All other markers are isozymes. Chromosome numbers are indicated at the top and the values along the side are map distances in cM. Linkage groups that did not show linkage with any of the mapped markers are indicated at the bottom of the figure.

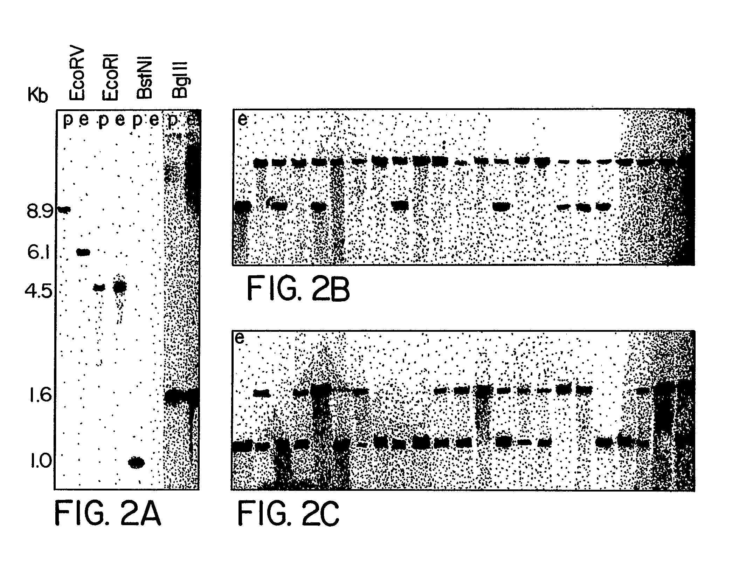

Figure 2(A) is a restriction enzyme survey of L. pennellii (p) and L. esculentum (e) probed with clone 3-41 (CD14). The values at left are the fragment sizes in kilobases. Figure 2(B) shows backcross progeny DNA ( L. pennellii as the recurrent parent) digested with EcoRV and probed with 3-41.

## Detailed Description of the Preferred Embodiments

This invention is based on the use of DNA restriction fragment length polymorphisms (RFLP's) to reveal differences in DNA taken from different plants.

DNA is isolated from a tomato plant and cut into fragments using a restriction enzyme by methods known in the art. A particular restriction enzyme cuts the DNA only at sites containing a specific nucleotide sequence, e.g. the restriction enzyme EcoRI cuts double stranded DNA only in the sequences GAATTC. Each restriction enzyme will cut the DNA of a particular plant into fragments of various lengths as specified by the distances between restriction enzyme recognition sites. Events such as insertion, deletion, and mutagenesis can alter the distance between restriction enzyme recognition sites.

As is known in the art, DNA fragments may be separated by size using gel electrophoresis. When it is desired to compare the DNA of two plants, the DNA samples of each are cut using the same restriction enzyme and the resulting fragments are separated according to size using electrophoresis. Many fragments from one variety may differ in length from their counterparts in another variety. Because any specific fragment represents a very small proportion of the total fragments, a sequence-specific probe must then be used to identify the specific homologous fragments in each DNA sample and permit comparison of fragment size.

This invention provides a unique set of cDNA probes for use in identifying corresponding DNA fragments from different tomato varieties. These probes were made, as described in the Examples hereof, by isolating mRNA from tomato variety VF36, preparation of a double-stranded copy DNA (cDNA) of the mRNA using reverse transcriptase, inserting the cDNA into bacterial plasmids, e.g. pBR322 and pUC8, and isolating clones harboring plasmids containing cDNA inserts (cDNA clones) larger than about 400-450 bp for further testing. All of these procedures are known in the art.

Individual cDNA clones were radioactively labeled to provide specific cDNA hybridization probes (probes). These cDNA probes will recognize substantially homologous sequences in any tomato variety. Tomato DNA fragments, after electrophoretic size separation, were transferred from the electrophoresis medium (typically an agarose gel) to a solid support (e.g. nitrocellulose or nylon membranes) such that the pattern resolved on the gel was preserved on the membrane. This membrane was incubated with radioactive probe during which time the probe hybridized to the specific corresponding DNA sequence. By observing the location of the probe on the membrane, it was possible to determine differences, or "length polymorphisms", between restriction fragments corresponding to the DNA of different tomato varieties. The procedure was repeated using different restriction enzymes and different probes until a number of restriction fragment length polymorphisms (RFLP's) by which the DNA of the two plants might be distinguished had been noted.

Only a few DNA fragments on the hybridization matrices showed homology to each cDNA probe, suggesting that the respective genes were present only once per haploid tomato genome. The majority of clones hybridized to different restriction fragments, indicating they each represented a different gene.

A set of unique cDNA probes, each identifying particular loci on the tomato genome, was derived. These cDNA probes are listed in Table 3 hereof, and are named both according to their original designation during the cloning process and according to their loci. The entire library comprising 60 cDNA probes in plasmids is on deposit according to Budapest Treaty requirements at the In Vitro International Depository of 7885 Jackson Road, Ann Arbor, Michigan 48103, deposited March 19, 1986, entitled "Tomato Nuclear cDNA Clones", under Accession No. IVI-10107. The cDNA clones are designated as follows: CD15; CD24; CD47; CD12; CD9; CD52; CD16; CD20; CD44; CD28; CD49; CD37; CD1; CD33; CD30; CD11; CD43; CD35; CD50; CD13; CD31; CD6; CD29; CD51; CD59; CD55; CD41; CD64; CD38; CD14; CD61; CD58; CD65; CD48; CD54; CD57; CD62; CD46; CD60; CD7; CD40;; CD21; CD56; CD45; CD34; CD27; CD4; CD3; CD8; CD25; CD42; CD5; 3-91; 3-167; 3-185; 3-48; 3-131; CD2; CD32 and CD39.

Using a number of different probes and restriction enzymes to analyze DNA from different plants, a DNA profile can be established for each plant, which is simply a compilation of the RFLP variants or alleles present in each variety. These profiles are used to differentiate plant varieties by comparing the RFLP's shown when one plant's DNA profile is compared with the DNA profile of the other.

This invention not only provides a method for identifying and distinguishing particular plant varieties, but because it was desired to use the cDNA clones in planned plant breeding programs, the position of the DNA sequence corresponding to each cDNA clone on the tomato chromosomes has been mapped. By the methods of this invention, individual traits or observable characteristics of tomato varieties can be linked with particular DNA markers, and this linkage used to transfer desired characteristics from one variety into another.

The cDNA clones are mapped as set forth below and as shown in Figure 1:

chromosome 1: CD15 - 22cM - CD24 - 17cM - CD47 - 4cM - CD12 - 27cM -CD9B and/or CD52A - 5cM - CD9A and/or CD16B - 5cM - CD16A and/or CD20 - 10cM - CD44 - 1cM - CD28;

chromosome 2: CD49B - 1cM - CD37 - 7cM - CD1 - 7cM - 3-91 - 15cM -CD33A - 9cM - CD30B - 14cM - 3-131 and/or CD11 - 25cM - CD43 - 4cM -CD35 - 6cM - CD9C - 27cM - CD30A and/or CD33B;

chromosome 3: CD50 - 2cM - 3-167 - 7 cM - CD13A and/or CD31B and/or CD6B and/or CD29C and/or CD52B - 17cM - CD51;

chromosome 4: CD59 - 5cM - CD49A - 39cM - CD55;

chromosome 5: CD41 - 9cM - CD64 - 8cM - CD31A - 10cM - CD38B;

chromosome 6: CD14 - 8cM - CD13B - 18cM - CD29B;

chromosome 7: CD61 - 3cM - CD58 and/or CD65 - 8cM - CD48 - 57cM -CD54 and/or CD57

chromosome 8: CD46 - 10cM - CD60 - 10cM - CD7 - 10cM -3-185 - 26cM - CD40 - 1cM - CD21A and/or CD16C;

chromosome 10: CD56 - 7cM - CD45 - 15cM - CD38A - 10cM - CD34B - 10cM - CD34A;

chromosome 12: CD21B - 1cM - CD27 - 7cM - CD4B and/or CD16D - 2cM -CD6A - 53cM - 3-48.

Unassigned Linkage Groups: 1) CD32A - 11cM - CD3 - 3cM - CD8

2) CD29D - 9cM - cD25B

3) CC42 - 8cM - CD25A

4) CD5 - 5cM - CD32B

Clone 3-48 hybridizes to all cab sites, but preferentially to cab-5.

Applicants' invention consists of the creation and identification of the specific clones, listed above, and also of the determination of the useful relationship of such clones to each other as indicated by their relative positions on the chromosome map. Other clones which may be prepared as hereinafter described and substituted for the above-listed clones, and which map to the same sites on the chromosomes, are equivalent to the above clones, and are designated herein by sequential numerical designations and listed below according to their relative positions on the chromosome map. It should be understood that clone 1 is functionally equivalent to CD15, clone 2 is functionally equivalent to CD24, etc., with each sequentially numbered clone defined by its ability to hybridize with chromosomal DNA 0-10 map units from the chromosomal DNA with which the named clones hybridize.

chromosome 1: clone 1 - 22cM - clone 2 - 17cM - clone 3 - 4cM - clone 4 - 27cM - clone 5 - 5cM - clone 6 - 5cM - clone 7 - 10cM - clone 8 -1cM - clone 9;

chromosome 2: clone 10 - 1cM - clone 11 - 7cM - clone 12 - 7CM -clone 13 - 15cM - clone 14 - 9cM - clone 15 - 14cM - clone 16 - 25cM -clone 17 - 4cM - clone 18 - 6cM - clone 19 - 27cM - clone 20;

chromosome 3: clone 21 - 2cM - clone 22 - 7cM - clone 23 - 17cM -clone 24;

chromosome 4: clone 25 - 5cM - clone 26 - 39cM - clone 27;

chromosome 5: clone 28 - 9cM - clone 29 - 8cM - clone 30 - 10cM -clone 31;

chromosome 6: clone 32 - 8cM - clone 33 - 18cM - clone 34;

chromosome 7: clone 35 - 3cM - clone 36 - 8cM - clone 37 - 57cM -clone 38

chromosome 8: - clone 41 - 10cM - clone 42 - 10cM -clone 43 - 10cM - clone 44 - 26cM - clone 45 - 1cM - clone 46;

chromosome 10: clone 47 - 7cM - clone 48 - 15cM - clone 49 - 10cM -clone 50 - 10cM - clone 51;

chromosome 12: clone 52 - 1cM - clone 53 - 7cM - clone 54 - 2cM -clone 55 - 53 cM - clone 56.

Unassigned Linkage Groups: 1) clone 57 - 11cM - clone 58 - 3cM -clone 59;

2) clone 60 - 9cM - clone 61;

3) clone 62 - 8cM - clone 63;

4) clone 64 - 5cM - clone 65.

Parents of two varieties (L. pennellii and L. esculentum) were crossbred and backcrossed, using L. pennelli as the recurrent parent, all as described in Example 2, and the segregations of DNA markers and known genetic markers were evaluated in the BC1 progeny. These known genetic markers include isozyme markers known to the art and described, for example in Tanksley, S.D. (1983) "Molecular Markers in Plant Breeding" Plant Mol. Biol. 1:1 pp. 3-8, incorporated herein by reference. The known tomato chromosome map including both the DNA markers of this invention, and previously known markers is shown in Figure 1. Using established methods of genetic linkage analysis, the segregation data were used to map the positions on the tomato genome of the cDNA markers. This involved calculating the percent of the progeny in which a cDNA marker cosegregated with a known marker. Genetic map distance is defined by the percent recombination observed between two loci. For example, if a given DNA marker co-segregated with a previously mapped marker 100% of the time, there would be 0% recombination and thus, the DNA marker is 0 cM (centiMorgans; map units) from the previously mapped marker. A 10% recombination rate would indicate a 10cM map distance from the previous marker, etc. A discussion of known methods of genetic mapping using RFLP's and practical applications thereof is given in Beckmann, J.S. and Soller, M. (1983), "Restriction fragment length polymorphisms in genetic improvement: methodologies, mapping and costs", Theor. and Appl. Genetics 67:35-43; and Soller, M. and Beckmann, J.S. (1983), "Genetic polymorphism in varietal identification and genetic improvement", Theor. and Appl. Genetics 67:25-33, both of which are incorporated herein by reference. See also Burr, B., Evola, S.D., Burr, F.A. and Beckmann, J.S. (1983), "The application of restriction fragment length polymorphisms to plant breeding", Genetic Engineering Principles and Methods (Setlow and Hollander, eds.) Vol. 5:45-49, also incorporated herein by reference.

The highly detailed map of this invention may be used to link phenotypic characteristics of a tomato plant with a particular marker or markers. To link an observable characteristic of a tomato plant with a cDNA marker clone or isozyme marker, the percent recombination of the observable trait with the marker is observed. Generally a recombination rate of less than 30% (indicating a map distance of 30 cM or less), is considered a useful linkage, with a recombination rate of 10% or less being preferred.

More specifically, in linking an observable trait or characteristic such as high solids with a cDNA clone of this invention, a tomato variety having the observable characteristic and another variety not having the observable characteristic are selected. (An observable characteristic is one observable by visual inspection, physical or chemical testing or other means and includes RFLP's observed through the use of additional probes.) The two parental varieties are crossed and the F1 progeny either selfed or backcrossed to provide a population of individuals. These individuals are segregating for now known DNA markers and observable trait(s) of interest. The DNA is extracted from the segregating individuals and treated with a restriction enzyme, separated by gel electrophoresis, and then transferred to a hybridization support. After hybridization with a cDNA probe, one may determine which corresponding parental DNA fragments are present in each individual, the parental DNA profile having been previously determined. The process is repeated with additional cDNA probes to establish which parental chromosomes or parts thereof are present in each individual segregating progeny. Preferably, all the cDNA probes are used so that maximum segregation information may be developed. Isoenzyme markers may also be used to provide additional information. Additional restriction enzymes may be used as necessary to determine polymorphisms.

Each segregating individual is also scored for the desired phenotypic characteristic (e.g. high solids content). Genetic linkage analysis is then used to analyze the segregation of the observable trait(s) with known markers. DNA markers from the donor parent which recombine with the observable characteristic more than 50% of the time are considered to be unlinked to the DNA sequences controlling the observable characteristic. Markers which show 30% or less recombination may be considered to be usefully linked to the trait(s). Markers which show 10% or less recombination with the observable characteristic(s), are considered to be closely linked to the genes controlling the trait(s).

In practice, a limited subset of markers capable of revealing the parental chromosomes composition of each parent may be applied first. A subsequent set of markers for a specific chromosome would subsequently be used. For example, if markers on chromosome 5 show moderate linkage to high percent solids, then additional markers on chromosome 5 are selected and tested, until a marker which shows the least recombination with high percent solids is identified. The recombination frequencies are then used to map the genes controlling the observable characteristic.

Specifically, the method for determining an heritable association between an observable characteristic of a tomato plant and a cDNA clone of this invention comprises: (a) selecting a tomato plant having the observable characteristic and another tomato plant not having the observable characteristic as parents for crossbreeding; (b) extracting DNA from the parent plants and subjecting said DNA to treatment with a restriction enzyme to obtain DNA fragments; separating the fragments from each plant on a neutral gel by electrophoresis and transferring the electrophoresed fragments to a solid support to obtain a hybridization matrix for each plant; (c) hybridizing the matrices of step (b) with a cDNA clone selected from the cDNA clones of this invention marking a chromosome or unassigned linkage group which might contain DNA corresponding to said observable trait (for example, prior experiments may indicate or rule out certain chromosomes as containing DNA controlling the trait); (d) comparing the hybridization pattern from each parent to determine the presence of polymorphisms; (e) repeating steps (b) - (d) utilizing at least one additional cDNA clone selected from those marking other chromosomes or unassigned linkage groups which might contain DNA corresponding to the said observable characteristic and/or using additional restriction enzymes as necessary until polymorphisms have been identified by which the parents may be distinguished from each other by at least one difference on each chromosome or unassigned linkage group which might contain DNA corresponding to the said observable characteristic; (f) crossing the parent plants to obtain an F1 generation and selfing or backcrossing the F1 generation to obtain a segregating generation containing individuals having the observable characteristic; (g) selecting and scoring a statistically significant number of segregating individuals, preferably at least about 40, for the percent presence of the observable characteristic; (h) extracting DNA from the segregating individuals of step (g) and following the procedures of steps (b) through (e) to obtain hybridized matrices for each individual showing polymorphisms when compared to the matrices of the parent not having the observable characteristic; (i) identifying cDNA clones which are located within 10 map units (10 cM) of genes conferring the observable characteristic; (j) if no such cDNA clones are identified, identifying cDNA clones which are located less than 30 map units (cM) from genes controlling the observable characteristic; (k) selecting additional cDNA clones from the cDNA clones of this invention, said cDNA clones having map positions spaced less than 30 map units (cM) from the cDNA clones of step (j), and hybridizing said clones with the DNA of segregating individuals of step (h); (l) identifying cDNA clones which are located within 10 map units (10cM) from genes controlling the observable characteristic; (m) if no such cDNA clones are identified, selecting additional cDNA clones from the cDNA clones of this invention, said cDNA clones having map positions spaced closer to previously tested cDNA clones having high percent identity than those having a lower percent identity, hybridizing said clones with the DNA of segregating individuals of step (h) and identifying cDNA clones which are located less than 10 map units from genes controlling the observable characteristic; (n) if no such cDNA clones are identified, repeating step (m) until such clones have been identified.

Isozyme and other markers can be utilized in addition to cDNA clones to link with heritable characteristics by correlating the presence or occurrence of each isozyme occurring in the parent having the observable characteristic with the percent presence of the observable characteristic in the segregating generation individuals.

DNA controlling the observable characteristic may be located on the chromosome map by placing it the number of map units from specific markers corresponding to its percent recombination with such markers.

The cDNA clones of this invention may be used to develop additional or substitute clones mapping to the same or contiguous regions (0 map units as defined by recombination rates) of the genome. For example, a phage or other clone library is probed with a cDNA clone of this invention and additional clones having sufficient homology to the cDNA clone to hybridize therewith are identified. Such newly-identified clones may be subcloned, utilizing restriction enzymes, and the newly-identified clones or fragments thereof are tested for their recombination rate with the previously mapped cDNA clones or linked characteristics. Those showing a 0-10% recombination rate are substitute clones equivalent to the named clones of this invention.

Hybridization conditions utilized in the procedures described herein are as set forth in Example 1. Hybridization conditions equivalent to those described herein may be employed by those skilled in the art utilizing well-known, published equations, for example as described in Nucleic Acid Hybridization, A Practical Approach, Hames, B.D. and Higgins, S.J., eds. (1985) IRL Press, Oxford, incorporated herein by reference.

Once a linkage between an observable characteristic and a marker has been established, any individual plant may be identified at an early stage of growth for the presence of the gene controlling that characteristic by noting the appearance of marker known to be linked with the characteristic. Consequently, a desired characteristic may be introgressed from one tomato variety into a second tomato variety having other desirable characteristics. For example, moving a trait such as Fusarium resistance from a wild tomato variety having otherwise undesirable fruit characteristics into a domestic variety can be facilitated by the availability of a marker which is closely linked to Fusarium resistance. As in the example provided above, a DNA marker which is closely linked to the genes controlling Fusarium resistance must first be identified. Segregating progeny resulting from a cross between the Fusarium resistance donor and the recipient are tested for the presence of this marker. Only those individuals carrying the marker need be saved. For example, if the desirable trait is due to one gene, 75% of the individuals could be discarded. In the process, additional markers are used to evaluate the extent to which each individual carrying the Fusarium resistance gene represents each parental genome. Those individuals which carry the Fusarium resistance marker and most closely represent the desirable parental genome would be retained for additional cycles of backcrossing with the desirable domestic variety. The backcrossing and screening is continued until individuals homozygous for the desired characteristic but otherwise having a DNA profile very close to the domestic parent are obtained. The key advantage of this approach is that it allows the breeder to converge upon the desirable genotype(s) rapidly with a minimum of field testing.

Specifically, a desired characteristic may be transferred from a tomato plant into a second tomato variety by: (a) linking the desired characteristic with a marker selected from the markers of this invention, isozyme markers of Fig. 1 or other markers; (b) probing DNA of the second tomato variety with cDNA of this invention to determine its DNA profile; (c) crossbreeding a tomato plant having the desired characteristic with a plant of the second tomato variety to obtain an F1 generation; (d) screening F1 individuals for being homozygous for the desired characteristic using the linked marker; (e) probing the DNA of the F1 individuals having the desired characteristic with cDNA of this invention to determine their DNA profiles; (f) selecting F1 individuals having the desired characteristic and otherwise having DNA profiles most similar to that of the parent of the second tomato variety for breeding; (g) backcrossing the selected individuals of step (f) with a parent of the second tomato variety to produce an BC1 generation; (h) screening BC1 individuals for the desired characteristic using the linked marker; (i) probing the DNA of the BC1 individuals having the desired characteristic with cDNA of this invention to determine their DNA profiles; (j) selecting BC1 individuals having the desired characteristic and otherwise having DNA profiles similar to that of the parent of the second tomato variety; and (k) repeating steps (g) - (i) with BC2 and subsequent generations until individuals homozygous for the desired characteristic and having the desired degree of similarity to the parent of the second tomato variety are produced.

An important application of the ability to use the clones of this invention to test genetic material for the presence or absence of DNA controlling particular traits is in the introgression of additional disease resistance genes into plant varieties already having other genes controlling resistance to the same disease. Overall resistance to a disease is greater in plants having several different genes controlling such resistance, but is is very difficult to test for the presence of multiple disease resistance genes in an actual plant because the presence of one resistance gene masks identification of resistance caused by additional genes. The use of the mapped markers of this invention to identify such additional resistance genes enables plant breeders to easily select for their presence and breed them into already resistant varieties.

The cDNA markers, chromosome map, and screening method of this invention are useful in a number of other applications which will be apparent to those skilled in the art. For example, these markers and methods may be used in estimation of ontcrossing rates, identification of sexual and parasexual hybrids, identification of breeding stock in perennial nurseries, measurement of genetic variability, determination of genetic purity of hybrid seed lots, identification of infringement of patents covering plant varieties and recombinant DNA, and identification of haploid-derived plants from anther culture.

The following examples illustrate the development and use of the cDNA clones and isozyme markers of this invention.

## Example 1: Number of loci per clone

cDNA Synthesis and Cloning. Polysomes were isolated from young leaves of the tomato variety VF36 of L. esculentum according to Kamalay and Goldberg (1980), "Regulation of structural gene expression in tobacco", Cell 19:935-946, and Jackson and Larkin (1976), "Influence of ionic strength, pH and chelation of divalent metals on isolation of polyribosomes from tobacco leaves", Plant Physiol. 57:5-10, both of which are incorporated herein by reference, and poly-adenylated mRNA was purified using oligo-dT cellulose according to the manufacturer's instructions (BRL). Double stranded, C-tailed cDNA was synthesized using the poly-A mRNA as template according to Murray, M.G., Hoffman, L.M. and Jarvis, N.P. (1983), "Improved yield of full-length phaseolin cDNA clones by controlling premature anticomplementary DNA synthesis", Plant Molec. Biol. 2:75-84 (Approach C), incorporated herein by reference. Approximately 20 ng of cDNA was annealed to 80 ng of dG-tailed PstI-cut pBR322 (BRL) and used to transform competent HB101 cells according to the method of Mandel, M. and Higa, A. (1970), "Calcium dependent bacteriophage DNA infection", J. Mol. Biol. 53:154, and Dagert, M. and Erlich, S.D. (1979), "Prolonged incubation in calcium chloride improves the competence of Escherichia coli cells", Gene 6:23, both of which are incorporated herein by reference.

Isolation of Plasmid DNA and Measurement of Insert Size. $Tet^R$ and $Amp^S$ colonies were grown in 1.5 ml cultures and the plasmid was isolated according to Birnboim, H.C. and Doly, J. (1979), "A rapid alkaline extraction procedure for screening recombinant plasmid DNA", Nucleic Acids Res. 7:1513. The plasmids were digested with PstI and run on 0.9% agarose gels with ØX174 DNA digested with HaeIII as size standards. Those bacteria with plasmids that had inserts greater than 400 bp were grown in large cultures. A similar procedure was used to isolate plasmid which was further purified by CsCl equilibrium density gradient centrifugation (Maniatis, T., Fritsch, E.F. and Sambrook, J., Molecular Cloning, Cold Spring Harbor Laboratory, New York, pp. 93-94).

Genomic DNA Isolation. Total plant cellular DNA was extracted from approximately 25 gms of fresh leaf tissue from individual plants. The procedure is a modification of that reported in Murray, M. and Thompson, W.F. (1980), "Rapid isolation of high molecular weight plant DNA", Nucl. Acids Res. 8:4321-4325, incorporated herein by reference. The tissue was homogenized in a blender with 150 ml of extraction buffer (100 mM Tris pH 8.0, 0.35 M Sorbitol, 5mM EDTA and 1.0% β-mercaptoethanol), filtered through cheesecloth/Miracloth (Calbiochem) and spun at 700 x g for 15 minutes in a JA10 rotor (Beckman). Each pellet was resuspended in 5 ml extraction buffer and transferred to a 50 ml screw cap centrifuge tube. The resuspension was adjusted to 1.0% CTAB (Hexadecyltrimethylammonium bromids, Sigma), 1 M NaCl, 25 mM EDTA, and then adjusted to 1.0% sarcosyl and heated to 60°C for 20 minutes. The lysate was extracted once with chloroform/octanol (24:1 v/v). The aqueous phase was mixed with two-thirds volume isopropanol to precipitate the DNA. The precipitate was washed in 76% ethanol, 10 mM ammonium acetate pH 7.0. The DNA was dissolved and further purified by CsCl/ethidium bromide centrifugation (Maniatis et al. 1982, supra).

Genetic Stocks. Hybrids were made between Lycopersicon esculentum cv. VF36 (LA916) and the wild species Lycopersicon pennellii (LA716). Both parents were highly inbred. F2 seeds were obtained by selfing the F1 hybrid.

Restriction digests, electrophoresis, blotting, hybridization and nick translation. DNA from each of the parental stocks and 46 F2 progeny described above was digested with the following restriction enzymes: DraI (New England BioLabs), EcoRI, EcoRV, HindIII, BstNI, SstI, PstI and Xba I (all from BRL). Digests were fun on 0.9% agarose gels and blotted onto Zetabind filters (AMF Cuno) according to Southern, E.M. (1975), "Deletion of specific sequences among DNA fragments separated by electrophoresis", J. Mol. Biol. 98:503-517. Filters were probed with each of 36 radio-labelled plasmids containing random cDNA inserts. Nick translation of plasmids followed the procedure of Rigby, P.W.J., Dieckman, M., Rhodes, C. and Berg, P. (1977), "Labeling deoxyribonucleic acid to high specific activity in vitro by nick translation with DNA polymerase I", J. Mol. Biol. 113:815-817, and yield specific activities of $10^8$ -$10^9$ dpm/$\mu$g. Filters were prehybridized for 6-12 hours at 65°C in a solution containing 5X SSC, 50mM $NaH_2PO4$ pH 7.4, 0.4% SDS, 5X Denhardt's, 2.5 mM EDTA, 5% dextran sulfate and 100$\mu$g/ml sheared, denatured salmon testes DNA. Labelled probe was then added at a rate of $10^6$ dpm/ml. Hybridizations took place at 65°C for 12-24 hours after which the filters were washed twice in 2X SSC, 0.1% SDS at 65°C for 20 minutes and twice in 1X SSC, 0.1% SDS at 65°C. Washed filters were placed against Kodak XAR-5 X-ray film for 12-72 hours at -70°C using DuPont Cronex Lightening-Plus intensifier screens.

Reconstruction Experiments. To obtain estimates of the number of genomic copies, several individual plasmids containing cloned tomato cDNA fragments were used in reconstruction experiments. Two 1$\mu$g aliquots of denatured plant DNA were spotted directly onto Zetabind using a slot-blot template according to the method of Rivin, C. (1986), "Analyzing genome variation in plants", Meth. Enzymol. 118:75-85. Onto the same Zetabind filter was also applied various quantities of denatured plasmid carrying specific tomato cDNA clones. Quantities of plasmid were added to approximate the amount expected for 0.5, 1 and 3 copies of the tomato insert in 1 $\mu$g of plant DNA. The amounts of plasmid used in the reconstructions were based on the reported C value of tomato of 0.74 pg = $7.14 \times 10^8$ bp (Galbraith, D.W., Harkins, K.R., Maddox, J.M., Ayres, N.M., Sharma, D.P., Firoozabady, E. (1983), "Rapid flow cytrometric analysis of the cell cycle in intact plant tissues", Science 220:1049-1051).

Each filter was then hybridized with the corresponding radiolabelled tomato insert. After hybridization, filters were washed to a stringency of 0.2 x SSC, 65°C. Derived autoradiographs were subjected to densitometric scanning. Densities corresponding to 0.5, 1 and 3 were plotted against the copy number to estimate the linear relationship between these two variables. The derived plots were subsequently used to estimate the number of copies of the sequence in the samples of plant DNA on the same film. A minimum of two and a maximum of four independent reconstruction estimates were made for each clone tested. The only exception was for 3-41 for which only one estimate was made. Means and mean standard errors were calculated for copy number estimation for each clone (Table 1). Note: clones are named herein according to their loci (CD numbers) as well as their assigned numbers, and these names are equivalent and interchangeable. A cross reference of names and loci is given in Table 2.

Table 1:  Clones used for copy number estimates.  Note:  Ave. no. RF's

(restriction fragments) based on digests with DraI, EcoRI,

EcoRV, HindIII, BstNI

| clone | size (bp) | locus | Ave. no. RF's | min.RF length (kb) | copy no. estimate X | Sx |
|-------|-----------|-------|---------------|--------------------|--------------------|-----|
| 3-17 | 1250 | CD2 | 1.00 | 3.7 | 0.7 | 0.10 |
| 3-14 | 400 | CD3 | 1.20 | 1.0 | 1.7 | 0.34 |
| 2-14 | 550 | CD5 | 1.00 | 1.0 | 2.1 | 0.87 |
| 2-24 | 625 | CD8 | 1.80 | 4.5 | 4.1 | 0.40 |
| 3-41 | 650 | CD14 | 1.75 | 3.1 | 1.9 | -- |
| 3-82 | 575 | CD15 | 1.40 | 1.0 | 2.1 | 0.40 |

Densitometric scans. Films derived from autoradiography were scanned on a BioMed soft laser scanning densitometer and areas corresponding to individual bands were integrated on an Apple IIe computer using BioMed software.

RESULTS

Size estimates (bp) were obtained for inserts from approximately 100 cDNA clones. Approximately 50% of the inserts were larger than 400 bp. Clones with inserts smaller than 400 bp were not further evaluated.

Of the clones with inserts larger than 400 bp, 36 were individually nick translated and hybridized to filters derived from restriction digests of DNA from the two parental tomato species. In most cases only a few of the genomic fragments showed homology to the probe suggesting that the respective genes were not present in high copy number in the plant genome. The majority of the clones hybridized to different restriction fragments indicating they were each representing a different gene. The only exceptions were clones 3-91 and 3-167 which gave identical hybridization profiles as did clones 3-185 and 3-131. The patterns of these sets of clones were virtually identical to profiles previously obtained for hybridizations with heterologous clones of the small subunit of ribulose bisphosphate carboxylase (RBCS) and the major chlorophyll a/b binding polypeptide (CAB) respectively (Vallejos, C.E., Tanksley, S.D. and Bernatzky, R. (1986), "Localization in the tomato genome of DNA restriction fragments containing sequences homologous to the rRNA (45s), the major chlorophyll a/b binding polypeptide and the ribulose bisphosphate carboxylase genes", Genetics 112:93-105). Hybridization of 3-91 and 3-167 to a cDNA RBCS clone from petunia (pSSU71) (Dunsmuir, P.S., Smith, S., Bedbrook, J.A. (1983), "A number of different nuclear genes for the small subunit of ribulose bisphosphate carboxylase are transcribed in Petunia", Nucleic Acids Res. 11:4177-4183) demonstrated that the two tomato clones were indeed for RBCS. Likewise clones 3-185 and 3-131 hybridized to a mungbean CAB cDNA clones (pMB123) (Thompson, W.F., Everett, M., Polans, N.O. and Jorgensen, R.A. (1983), "Phytachrome control of RNA levels in developing pea and mung bean leaves", Planta 158:487-500) confirming their suspected identity.

Number of loci per clone . The parental surveys were used to select restriction enzymes which would give profiles with the least number of hybridizing fragments in common between the two parents using a given clone as a probe. Filters containing F2 DNA (from 46 individual plants) digested with these selected enzymes were then probed with the labelled plasmid to detect segregation of restriction fragments. Fragments which co-segregated were considered to belong to the same locus whereas fragments which did not co-segregate were considered to belong to different loci. Accordingly, locus designations were given to the detected loci (Table 2). Segregation ratios for each locus are also given in Table 2.

For 28 of the 34 unique clones evaluated, more than 95% of the genomic hybridization signal (as determined by densitometric scans of autoradiographs) could be accounted for by fragments which were segregating (Table 2). With four more of the clones more than 85% of the signal could be accounted for. For one clone, 3-31, only 60% of the signal could be accounted for.

Based on the genetic analysis performed on the 34 clones, it was possible to determine the number of loci corresponding to each cDNA clones (Table 2). Fifty-three percent of the clones (18 clones) hybridized to genomic fragments belonging to single chromosomal loci. Thirty-two percent (11 clones) corresponded to two loci. For the remainder, two clones (6%) corresponded to 3 loci, two to 4 loci (6%) and one to 5 separate loci (3%). None of the clones belonged to gene families arranged in more than 5 loci.

Multilocus clones. For clones homologous to more than one locus, the distribution of genomic hybridization signal (from autoradiographs) was often not equal amon all loci (Table 2). For example, clone 3-87 hybridized to two independent loci, CD13A and CD13B. However, CD13A accounted for approximately five times more hybridization signal then CD13B (Table 2). When genomic DNA was digested with 4 different restriction enzymes and hybridized to 3-87, the sizes of genomic DNA fragments corresponding to CD13A and CD13B were often relatively short (less than 3.0 kb). Given that 3-87 is approximately 750 bp long, it seems doubtful that there would be room for more than 1 or 2 copies of the structural gene in each locus. This fact suggests that the different hybridization signal between CD13A and CD13B is not due to a difference in the number of copies of the gene in each locus, but instead it is probably a result of significant sequence divergence of the structural sequences contained in the two loci.

Of the other ten clones showing homology to two loci (2-23, 3-6, 3-93, 3-99, 3-140, 3-136, 3-159, 3-190, 3-176, 3-275), seven gave similar results as those with 3-87 in which one locus accounts for a much higher proportion of the hybridization signal (Table 2). For some of these the possibility cannot be ruled out that the locus giving the greatest hybridization signal possesses more than 1 copy of the gene.

Three clones (3-91, 3-167, 2-21) were from gene families found in three independent loci. 3-91 and 3-167 turned out to be clones for the small subunit of ribulose bisphosphate carboxylase as determined by hybridization to a heterologous clone for this gene from petunia (pSSU71, Dunsmuir et al. 1983, supra). The hybridization profiles of 3-91 and 3-167 matched very closely those reported for hybridization of tomato genomic DNA to the petunia RBCS clone (Vallejos et al. 1986, supra). In fact, no new hybridizing fragments were revealed with the homologous tomato probes--a fact which suggests that the number of loci reported in the previous study (3 loci), using the heterologous petunia clone, is probably correct.

Clone 2-21 hybridized with 3 genetic loci, CD9A, CD9B and CD9C, with roughly equal intensity. This result may indicate that either: 1) The duplication event leading to 3 loci was a recent one and sufficient time has not elapsed for significant sequence divergence; 2) These genes are under strong selection pressure which reduced the rate of divergence; 3) The genes are co-evolving through some as yet unknown mechanism. The discovery that pepper (Capsicum annuum), which belongs to a related genus in the family Solanaceae, has only a single locus homologous to 2-21 supports the first explanation.

Two clones (3-95 and 3-155) were found to each correspond to four loci. For 3-95, three of the loci (CD16A, CD16B, CD16C) yielded roughly equal hybridization intensities whereas the fourth locus, CD16C, gave a hybridization signal approximately 1/3 than the other loci (Table 2). Loci corresponding to 3-155 were of varying hybridization intensities suggesting a different number of genes per locus or considerable divergence among the loci.

The only clones to hybridize to more than four loci were 3-185 and 3-131. Hybridization to a mungbean clone of the major chlorophyll A/B binding polypeptide (CAB) (pMB123, Thompson et al. 1983, supra), revealed that these clones were both derived from CAB gene(s). The genetics of tomato CAB sequences, as determined by genomic hybridizations with the heterologous clnone from mung bean, has recently been published (Vallejos et al. (1986), supra). Hybridization patterns derived from the two tomato cDNA clones reported here yield similar results as those with the mung bean probe, reflecting a high degree of conservation in the coding region of this gene.

Table 2: Characteristics of loci in the tomato genome corresponding to random cDNA clones. Unless otherwise noted, $X^2$ is for goodness-of-fit to 1:2:1 ratio. Percent hybridization signal was determined from densitometric scans of autoradiographs (see Materials and Methods).

| clone | size (bp) | no. loci detected | locus symbol(s) | % hybrid-ization signal | e/e | e/p | p/p | $X^2$ | |
|-------|-----------|-------------------|-----------------|-------------------------|-----|-----|-----|-------|---|
| 2-13 | 2450 | 1 | CD1 | 100 | 5 | 25 | 15 | 5.00 | |
| 3-17 | 1250 | 1 | CD2 | 100 | 11 | 20 | 12 | 0.25 | |
| 3-14 | 400 | 1 | CD3 | 100 | 8 | 23 | 14 | 1.62 | |
| 2-23 | 600 | 2 | CD4A | 52 | 7 | 27 | 12 | 2.48 | |
| | | | CD4B | 48 | 7 | 25 | 13 | 2.16 | |
| 2-14 | 550 | 1 | CD5 | 100 | 6 | 25 | 13 | 3.05 | |
| 3-6 | 900 | 2 | CD6A | 95 | 7 | 24 | 13 | 2.00 | |
| | | | CD6B | 5 | | 30 | 12 | 0.26 | A |
| 3-13 | 600 | 1 | CD7 | 100 | 10 | 20 | 13 | 0.63 | |
| 2-24 | 625 | 1 | CD8 | 100 | 8 | 24 | 14 | 1.65 | |
| 2-21 | no site | 3 | CD9A | 35 | 13 | 17 | 12 | 1.57 | |
| | | | CD9B | 35 | 13 | 17 | 12 | 1.57 | |
| | | | CD9C | 30 | 2 | 25 | 15 | 9.57 | ** |
| 3-50 | 625 | 1 | CD11 | 100 | 14 | 24 | 4 | 5.62 | |
| 3-31 | 550 | 1 | CD12 | 60 | 5 | 24 | 15 | 4.91 | |
| 3-87 | 750 | 2 | CD13A | 83 | 4 | 30 | 12 | 7.04 | * |
| | | | CD13B | 17 | 9 | 24 | 13 | 0.78 | |
| 3-41 | 650 | 1 | CD14 | 100 | 9 | 22 | 11 | 0.29 | |
| 3-82 | 575 | 1 | CD15 | 100 | 8 | 17 | 18 | 6.53 | ** |
| 3-95 | 750 | 4 | CD16A | 33 | 15 | 19 | 10 | 1.95 | |
| | | | CD16B | 25 | 15 | 16 | 13 | 3.45 | |
| | | | CD16C | | 31 | 35 | 11 | 0.03 | A |
| | | | CD16D | 8 | 31 | 13 | | 0.48 | A |
| 3-109 | 700 | 1 | CD20 | 100 | 15 | 21 | 10 | 1.43 | |
| 3-93 | 575 | 2 | CD21A | 70 | 11 | 23 | 12 | 0.04 | |
| | | | CD21B | 20 | 7 | 21 | 16 | 3.77 | |
| 3-111 | 575 | 1 | CD24 | 100 | 5 | 19 | 16 | 6.15 | * |
| 3-99 | 550 | 2 | CD25A | 80 | 8 | 28 | 8 | 3.27 | |
| | | | CD25B | 20 | 9 | 20 | 13 | 0.86 | |
| 3-132 | 550 | 1 | CD27 | 100 | 7 | 23 | 16 | 3.52 | |
| 3-152 | 500 | 1 | CD28 | 100 | 14 | 23 | 9 | 1.09 | |
| 3-155 | 650 | 4 | CD29A | 58 | 12 | 17 | 15 | 2.68 | |
| | | | CD29B | 15 | 6 | 29 | 9 | 4.86 | |
| | | | CD29C | 7 | 6 | 26 | 12 | 3.09 | |
| | | | CD29D | 7 | 9 | 22 | 13 | 0.73 | |
| 3-140 | 900 | 2 | CD30A | 88 | 6 | 28 | 11 | 3.80 | |
| | | | CD30B | 12 | 3 | 18 | 21 | 16.53 | ** |
| 3-126 | 775 | 2 | CD31A | 95 | 9 | 24 | 10 | 0.63 | |
| | | | CD31B | 5 | 2 | 15 | 5 | 3.37 | |
| 3-159 | 700 | 2 | CD32A | 63 | 4 | 26 | 16 | 7.04 | * |
| | | | CD32B | 37 | 6 | 24 | 9 | 2.54 | |

Table 2 (cont.)

| clone | size (bp) | no. loci detected | locus symbol(s) | % hybrid-ization signal | e/e | e/p | p/p | $\chi^2$ | |
|---|---|---|---|---|---|---|---|---|---|
| 3-190 | 550 | 2 | CD33A | 90 | 5 | 20 | 19 | 9.27 | ** |
| | | | CD33B | 10 | 4 | 11 | 7 | 0.82 | |
| 3-176 | 600 | 2 | CD34A | 28 | 3 | 25 | 17 | 9.27 | ** |
| | | | CD34B | 60 | 3 | 28 | 15 | 8.43 | * |
| 3-231 | 1300 | 1 | CD35 | 100 | 6 | 21 | 15 | 3.86 | |
| 3-288 | 1125 | 1 | CD37 | 100 | 8 | 23 | 15 | 2.13 | |
| 3-275 | 775 | 2 | CD38A | 20 | 2 | 26 | 14 | 9.24 | ** |
| | | | CD38B | 80 | 10 | 23 | 9 | 0.43 | |
| 3-287 | 1250 | 1 | CD39 | 100 | 9 | 20 | 16 | 2.73 | |
| 3-249 | 575 | 1 | CD40 | 100 | 12 | 22 | 12 | 0.09 | |
| (3-91, | 650 | 3 | Rbc-1=A | 8 | 6 | 24 | 15 | 3.80 | |
| 3-167) | 400 | | Rbc-2=B | 43 | 5 | 28 | 12 | 4.87 | |
| | | | Rbc-3=C | 48 | 5 | 25 | 14 | 4.50 | |
| (3-185, | 625 | 5 | Cab-1=A | 62 | 5 | 26 | 14 | 4.69 | |
| 3-131) | 600 | | Cab-2=B | 5 | 12 | 21 | 12 | 0.20 | |
| | | | Cab-3=C | 22 | 8 | 25 | 10 | 1.33 | |
| | | | Cab-4=D | 8 | 15 | 23 | 6 | 3.77 | |
| | | | Cab-5=E | 3 | 8 | 32 | 4 | 9.82 | ** |

A

Allelic L. pennellii restriction fragment not found. F2 segregation data is for presence (1st number) or absence (2nd number) of L. esculentum allele. $\chi^2$ is for goodness-of-fit to 3:1 ratio

*

Significant deviation at 0.05 level

**

Significant deviation at 0.01 level

Estimate of locus size. Genomic DNA of the inbred line L. Esculentum cv VF36 was digested with the following restriction enzymes: DraI, EcoRI, EcoRV, HindIII, BstNI. Derived Southern blots were then probed with each of 6 clones found to correspond to single loci: 3-17 (CD2), 3-14 (CD3), 2-14 (CD5), 2-24 (CD8), 3-41 (CD 14), 3-82 (CD15). Most of the clones hybridized to only a single restriction fragment. The average number and the minimum length of hybridizing restriction fragment(s) for any given digest are given in Table 1. The minimum restriction fragment length was considered to represent an estimate of the size of the coding portion of the genetic locus. The results from this analysis indicated that the loci under consideration are large enough to contain only 1 or a few copies of the gene (Table 1), a conclusion supported by genomic reconstruction experiments (see below).

*Gene copy number per locus.* Genomic reconstruction experiments (see Materials and Methods) were conducted on the same clones listed above in an attempt to independently estimate how many copies of the gene are present in each. Five of the six clones gave copy estimates corresponding to 1 or 2 copies of the gene per locus (Table 1). Clone 2-24 (CD8) gave a higher estimate (4 copies). This clone was also found to hybridize on genomic digests with most restriction enzymes to more than one fragment. From the segregation data it is known that all of those fragments belong to the same locus. This information coupled with the higher copy number estimate suggests that this locus may contain several copies of the gene.

Example 2: Map loci

*Plant materials.* Segregating populations were obtained from crosses between inbred lines of Lycopersicon esculentum (LA1500) and L. pennelli(LA716). A backcross to L. pennellii (staminate parent) and a BC1 population of 46 plants each were analyzed. The two parental lines had allelic differences at the following isozyme loci: Aco-1, Aco-2, Aps-1, Aps-2, Pgm-1, Pgm-2, 6Pgdh-2, Skdh-1, Prx-1, Prx-2, Prx-4, Pgi-1, Tpi-2, sod-1, Sod-2, Est-3, Est-7, and Est-8. Except for Sod-1, Sod-2 and Est-8, all of the isozymes had been previously mapped (Tanksley, S.D. (1985) "Enzyme coding genes in tomato (Lycopersicon esculentum)", Isozyme Bull. 18:43-45, incorporated herein by reference. Linkage analysis of isozyme markers in relation to genomic sequences homologous to the major chlorophyll a/b binding protein (Cab ), the small subunit of ribulose bisphosphate carboxylase (Rbcs ) and the large subunit of ribosomal RNA (R45s) have been reported by Vallejos et al. (1986) supra, incorporated herein by reference, and in this work provided additional segregating loci with which to map the random cDNA clones.

Chromosomes 5, 9, and 11 were not represented by any isozymes that were segregating in these populations. To test for cDNA markers on chromosome 5, progeny were used from a cross (I. esculentum x L. pennellii) segregating for the morphological markers af and tf which had been previously assigned to this chromosome as described by Rick, C.M. (1980), "Tomato linkage survey", Rep. Tomato Genet. Coop. 30:2-17.

*DNA extractions:* DNA was extracted from plant material by a modified procedure of Murray and Thompson (1980), supra, as described in Example 1.

*mRNA isolation and cDNA cloning:* Total polyadenylated leaf mRNA was isolated and cloned into pBR322 as described in Example 1.

*Restriction digests, electrophoresis, Southern blotting and hybridizations:* DNA from parental lines and their progeny were each digested with the following restriction enzymes: Dral, HindIII (New England BioLabs), EcoRI, EcoRV, SstI, PstI and XbaI (Bethesda Research Labs). Electrophoresis of plant DNA, Southern blotting, hybridization and nick translation of probes were as described in Example 1.

*cDNA screening:* Random cDNA clones were screened for insert size and those clones with inserts greater than 450 base pairs (bp) were used as probes. The parental DNAs were digested with various restriction enzymes, electrophoresed, blotted and probed with the cDNAs to determine which enzymes produced fragment length polymorphisms (Figure 2a). The appropriate enzymes were then used on progeny DNA. For the backcross progeny, L. pennelliiwas used as the recurrent parent such that only the L. esculentum fragments were segregating as present or absent. The F2 progeny segregated into the expected three genotypes for each locus with occasional skewing towards L. pennellii homozygotes.

*Linkage analysis:* Two-way contingency tests for independent assortment of isozyme markers and cDNA sequences based on chi-square analysis were accomplished using the SAS Proc Freq software program on an Amdahl 470/V5 computer. Loci were considered to be independent if the chi-square probability level was greater than 0.05. Map distances (cM) were calculated based on the maximum likelihood equations of Allard, R.W. (1954), "Formulas and tables to facilitate the calculation of recombination values in heredity", Hilgardia 24:235-278, incorporated herein by reference.

Table 3: Summary of cDNA clones, the number of loci per clone and their assigned chromosomes.

| Clone | Locus designation | Chromosomal assignment | Clone | Locus designation | Chromosomal assignment |
|---|---|---|---|---|---|
| 2-13 | CD1 | 2 | 3-176 | CD34A | 10 |
| 3-17 | CD2 | | | CD34B | 10 |
| 3-14 | CD3 | | 3-231 | CD35 | 2 |
| 2-23 | CD4A | | 3-288 | CD37 | 2 |
| | CD4B | 12 | 3-275 | CD38A | 10 |
| 2-14 | CD5 | 10 | | CD38B | 5 |
| 3-6 | CD6A | 12 | 3-287 | CD39 | |
| | CD6B | 3 | 3-249 | CD40 | 8 |
| 3-13 | CD7 | 8 | 3-217 | CD41 | 5 |
| 2-24 | CD8 | | 3-218 | CD42 | |
| 2-21 | CD9A | 1 | 2-17 | CD43 | 2 |
| | CD9B | 1 | 3-4 | CD44 | 1 |
| | CD9C | 2 | 3-10 | CD45 | 10 |
| 3-50 | CD11 | 2 | 3-16 | CD46 | 8 |
| 3-31 | CD12 | 1 | 3-27 | CD47 | 1 |
| 3-87 | CD13A | 3 | 3-38 | CD48 | 7 |
| | CD13B | 6 | 3-44 | CD49A | 4 |
| 3-41 | CD14 | 6 | | CD49B | 2 |
| 3-82 | CD15 | 1 | 3-81 | CD50 | 3 |
| 3-95 | CD16A | 1 | L4 | CD51 | 3 |
| | CD16B | 1 | L1 | CD52A | 1 |
| | CD16C | 8 | | CD52B | 3 |
| | CD16D | 12 | L3 | CD54 | 7 |
| 3-109 | CD20 | 1 | L2 | CD55 | 4 |
| 3-93 | CD21A | 8 | L5 | CD56 | 10 |
| | CD21B | 12 | L9 | CD57 | 7 |
| 3-111 | CD24 | 1 | L17 | CD58 | 7 |
| 3-99 | CD25A | | L16 | CD59 | 4 |
| | CD25B | | L13 | CD60 | 8 |
| 3-132 | CD27 | 12 | L18 | CD61 | 7 |
| 3-152 | CD28 | 1 | L20 | CD62 | 7 |
| 3-155 | CD29A | | L22 | CD64 | 5 |
| | CD29B | 6 | L10 | CD65 | 7 |
| | CD29C | 3 | | | |
| | CD29D | | 3-91, 3-167 | Rbc-1 | 2 |
| 3-140 | CD30A | 2 | | Rbc-2 | 3 |
| | CD30B | 2 | | Rbc-3 | 2 |
| 3-126 | CD31A | 5 | | | |
| | CD31B | 3 | 3-131, 3-185 | Cab-1 | 2 |
| 3-159 | CD32A | | | Cab-2 | 8 |
| | CD32B | 10 | | Cab-3 | 3 |
| 3-190 | CD33A | 2 | | Cab-4 | 7 |
| | CD33B | 2 | 3-48 | Cab-5 | 12 |

A sample restriction enzyme survey for clone 3-41 (CD14) is shown in Figure 2a. Four different restriction enzymes were used to digest the two parental DNAs. As seen in this figure, the restriction enzymes Eco RV and BstNI are suitable to distinguish the two parents (the BstNI fragment(s) of L. esculentum were small and ran off the gel). Digests of BC and F2 progeny DNA with EcoRV are shown in Figures 2b and 2c. The backcross progeny are only segregating for the L. esculentum (the non-recurrent parent) and hybrid genotypes whereas all three genotypes are displayed in the F2.

An example of the linkage analysis can be seen in Table 4. In this table, the pairwise linkage values for markers on part of chromosome 2 are presented. The chi-square probability levels for tests of independence are also indicated. Although the deduced gene order could usually be derived from pairwise linkage comparisons alone, three point linkage tests were always employed to confirm the linear order of linked markers.

Table 4: Linkage relationships among markers on part of chromosome 2. Values shown are in cM and the associated $X^2$ are in parentheses. $X^2$ values corresponding to a probability > 0.05 are considered non-significant (NS).

| | Cab-1 | Est-7 | Prx-2 | CD35 | CD9C | Rbcs-3 | CD30A |
|---|---|---|---|---|---|---|---|
| Cab-1 | --- | 12.8 (0.001) | 23.2 (0.007) | 23.5 (0.001) | NS (0.211) | NS (0.727) | NS (0.832) |
| Est-7 | | --- | 8.5 (0.001) | 14.2 (0.001) | 25.9 (0.002) | 25.9 (0.047) | NS (0.147) |
| Prx-2 | | | --- | 10.0 (0.001) | 15.0 (0.001) | 18.8 (0.001) | NS (0.069) |
| CD35 | | | | --- | 6.4 (0.001) | 13.1 (0.001) | 24.7 (0.015) |
| CD9C | | | | | --- | 13.0 (0.001) | 21.7 (0.024) |
| Rbcs-3 | | | | | | --- | 13.8 (0.001) |

Deduced gene order:

| Cab-1 | Est-7 | Prx-2 | CD35 | CD9C | Rbc-3 | CD30A |
|---|---|---|---|---|---|---|
| --:-----------:---------:----------:------:---------:-------------:--- |
| 13 | 8 | 10 | 6 | 13 | 14 | |

cDNA markers have been mapped to most of the tomato chromosomes (Figure 1). Chromosomes 1, 2 and 12 are very well marked. For example, the distance between the markers on chromosome 2, including isozymes and DNA fragments, averages approximately 8 cM and these markers cover a total map distance of 115 cM. The total presently-mapped isozyme and DNA markers cover approximately 779 cM between markers and have the ability to detect linkage for at least an additional 10 cM beyond each end. These markers, then are able to detect linkage over 88% of the tomato genome based on an estimate of 1200 cM for the entire genome.

Several cDNA markers were not assigned to chromosomal positions since they were not linked to any of the mapped markers used in this study. Unassigned linkage groups are indicated at the bottom of Figure 1. Individual clones that did not show any linkage are listed in Table 3 and are identified by lack of chromosomal assignment. Chromosome 5 did not have any segregating isozyme markers associated with it. However, a stock with morphological mutants that have been assigned to this chromosome was employed. DNA was isolated from 20 individuals of an F2 population (L. esculentum x L. pennellii) that was segregating for two markers on chromosome 5 (af and tf) and the various unassigned linkage groups were tested against these markers. cDNA marker CD41 showed tight linkage with tf such that the linkage group comprised of CD38B, CD31A and CD41 could be assigned to chromosome 5. Since reference markers were not available on chromosomes 9 and 11, it is likely that some, if not all, of the unmapped markers reside on these chromosomes.

Most of the cDNA fragments were found to correspond to single chromosomal locations. A few of the clones, however, are represented by sequences arranged as two, three or four loci (Table 3). Of these multi-locus clones, the majority show genetic independence among members, that is, the loci are dispersed throughout the genome. For example, a restriction enzyme survey of parental DNAs probed with 3-275 (CS38A and B) as well as progeny DNA from the F2 population shows this clone hybridizes to two fragments in both parental DNAs, and as indicated by the progeny DNA, the two fragments represent two loci which are genetically independent. There are a few exceptions to the independence of multi-locus clones. Two clones, 2-21 (CD9) and 3-176 (CD34), both of which hybridized to fragments at two loci, have linkage values of 5 and 10 cM between duplicate loci, respectively (Figure 1, chromosome 1 and 10). A number of other multi-locus clones have members that map to the same chromosome though they are more than 50 cM apart (Figure 1). As can be seen on chromosome 2, clones that represent CD30, CD33 and Rbcs are linked in two clusters, suggesting the duplication of a rather large segment.

The foregoing Examples are intended to illustrative rather than limiting to the scope of these inventions.

As discussed above, the markers of this invention were mapped using statistical methods. Variations in map distances from 5 to 10 cM from those given in Figure 1 are within the scope of equivalents to this invention.

## Claims

1. A cDNA library corresponding to tomato DNA comprising at least two clones selected from the group consisting of CD15; CD24; CD47; CD12; CD9; CD52; CD16; CD20; CD44; CD28; CD49; CD37; CD1; CD33; CD30; CD11; CD43; CD35; CD50; CD13; CD31; CD6; CD29; CD51; CD59; CD55; CD41; CD64; CD38; CD14; CD61; CD58; CD65; CD48; CD54; CD57; CD62; CD46; CD60; CD7; CD40; CD21; CD56; CD45; CD34; CD27; CD4; CD3; CD8; CD25; CD42; CD5; CD32; 3-91; 3-167; 3-185; 3-48; 3-131.

2. The cDNA library of claim 1 wherein said two clones are selected from the same chromosome.

3. The cDNA library of claim 1 containing all the clones listed in claim 1.

4. A cDNA clone selected from the group consisting of CD15; CD24; CD47; CD12; CD9; CD52; CD16; CD20; CD44; CD28; CD49; CD37; CD1; CD33; CD30; CD11; CD43; CD35; CD50; CD13; CD31; CD6; CD29; CD51; CD59; CD55; CD41; CD64; CD38; CD14; CD61; CD58; CD65; CD48; CD54; CD57; CD46; CD60; CD7; CD40; CD21; CD56; CD45; CD34; CD27; CD4; CD3; CD8; CD25; CD42; CD5; 3-91; CD32; 3-167; 3-185; 3-48; 3-131.

5. A plasmid containing a cDNA clone of claim 4.

6. The plasmid of claim 5 comprising pBR322 or pUC8.

7. A chromosome map useful for determining the original position or positions of a tomato DNA fragment on the tomato chromosome comprising a representation of at least one chromosome or unassigned linkage group with markers spaced therealong in sequential order with spacing given in map units (cM) as follows:

chromosome 1: CD15 - 22cM - CD24 - 17cM - CD47 - 4cM - CD12 - 27cM - CD9B and/or CD52A - 5cM - CD9A and/or CD16B - 5cM - CD16A and/or CD20 - 10cM -CD44 - 1cM - CD28;

chromosome 2: CD49B - 1cM - CD37 - 7cM - CD1 - 7cM - 3-91 - 15cM - CD33A -9cM - CD30B - 14cM - 3-131 and/or CD11 - 25cM - CD43 - 4cM - CD35 - 6cM -CD9C - 27cM - CD30A and/or CD33B;

chromosome 3: CD50 - 2cM - 3-167 - 7cM - CD13A and/or CD31B and/or CD6B and/or CD29C and/or CD52B - 17cM - CD51;

chromosome 4: CD59 - 5cM - CD49A - 39cM - CD55;

chromosome 5: CD41 - 9cM - CD64 - 8cM - CD31A - 10cM - CD38B;

chromosome 6: CD14 - 8cM - CD13B - 18cM - CD29B;

chromosome 7: CD61 - 3cM - CD58 and/or CD65 - 8cM - CD48 - 57cM - CD54 and/or CD57;

chromosome 8: CD46 - 10cM - CD60 - 10cM - CD7 - 10cM - 3-185 - 26cM - CD40 -1cM - CD21A and/or CD16C;

chromosome 10: CD56 - 7cM - CD45 - 15cM - CD38A - 10cM - CD34B - 10cM -CD34A;

chromosome 12: CD21B - 1cM - CD27 - 7cM - CD4B and/or CD16D - 2cM - CD6A - 53cM - 3-48.

Unassigned Linkage Groups: 1) CD32A - 11cM - CD3 - 3cM - CD8

2) CD29D - 9cM - CD25B

3) CD42 - 8cM - CD25A

4) CD5 - 5cM - CD32B

8. The map of claim 7 which also comprises isozyme markers, as follows:

chromosome 1: Idh-1 - 18cM - Prx-1 - 21cM - CD15 - 18cM - sdkh-1 - 4cM - CD24 - 17cM - CD47 - 4cM - CD12 - 27cM - CD9B and/or CD52A - 5cM - CD9A and/or CD16B - 5cM - CD16A and/or CD20 and/or Act-3 - 10cM - CD44 - 1cM - CD28 -18cM - Est-3;

chromosome 2: R45S and/or CD49B - 1cM - CD37 - 7cM - CD1 - 7cM - Rbcs-1 and/or 3-91 - 15cM - CD33A - 9cM - CD30B - 14cm - Cab-1 and/or 3-131 and/or CD11 - 11cM - Est-7 - 8cM - Prx-2 - 6cM - CD43 - 4cM - CD35 - 6cM - CD9C -13cM - Rbcs-3 - 14cM - CD30A and/or CD33B;

chromosome 3: Cab-3 - 12cM - Pgm-1 - 18cM - CD50 - 2cM - Rbcs-2 and/or 3-167 - 7cM - CD13A and/or CD31B and/or CD6B and/or CD29C and/or Act-4 and/or CD52B - 17cM - CD51;

chromosome 4: 6Pgdh-1 - 11cM - CD59 - 5cM - CD49A - 11cM - Act-1 and/or Tpi-2 - 15cM - Pgm-2 - 4cM - Adh-1 - 9cM - Act-10 and/or CD55;

chromosome 5: CD41 - 9cM - CD64 - 8cM - CD31A - 10cM - CD38B;

chromosome 6: CD14 - 8cM - CD13B - 7cM - Aps-1 - 11cM - CD29B;

chromosome 7: CD61 - 3cM - CD58 and/or CD65 - 8cM - CD48 - 16cM - Got-3 -7cM - Aco-2 - 18cM - Got-2 - 7cM - Cab-4 - 9cM - CD54 and/or CD57;

chromosome 8: CD46 - 10cM - CD60 - 10cM - CD7 - 10cM - Cab-2 and/or 3-185 -12cM - Aps-2 - 14cM - CD40 - 1cM - CD21A and/or CD16C;

chromosome 10: CD56 - 7cM - CD45 - 10cM -EstB - 10cM - CD34A and/or Act-6;

chromosome 12: CD21B - 1cM - 6Pgdh-2 and/or CD27 - 7cM - CD4B and/or CD16D - 2cM - CD6A - 3cM - Pgi-1 - 40cM - Aco-1 - 10cM - Cab-5 and/or 3-48.

Unassigned Linkage Groups: (1) - CD32A - 11cM - CD3 - 3cM - CD8;

(2) - Sod-1 and/or Act-5 - 7cM - CD29D -9cM - CD25B;

(3) - CD42 - 8cM - CD25A;

(4) - Act-2 - 6cM - CD5 - 5cM - CD32B.

9. The map of claim 7 or claim 8 in combination with at least one of the cDNA clones named therein.

10. A method for linking an observable characteristic of a tomato plant with a cDNA marker clone of claim 7 comprising comparing the percent recombination of the observable characteristic with the cDNA marker clones of claim 7 and designating the cDNA marker clone having the lowest percent recombination with the observable characteristic as being linked with the observable characteristic.

11. The method of claim 10 wherein the cDNA clones displaying less than 30% recombination with the observable characteristic are designated as linked with said characteristic.

12. A method for identifying a substitute DNA marker comprising probing a DNA library with a cDNA marker of claim 7 to identify library DNA substantially homologous to said cDNA marker, observing the recombination rate of said library DNA or a fragment thereof with said cDNA marker or an observable characteristic linked thereto, and designating library DNA having a 0-10% recombination rate as a substitute DNA marker.

13. A method for determining an heritable association between an observable characteristic of a tomato plant and a cDNA clone of claim 7 comprising:

(a) selecting a tomato plant having the observable characteristic and another tomato plant not have the observable characteristic as parents for crossbreeding;

(b) extracting DNA from the parent plants and subjecting said DNA to treatment with a restriction enzyme to obtain DNA fragments; separating the fragments from each plant on a neutral gel by electrophoresis and transferring the electrophoresed fragments to a solid support to obtain a hybridization matrix for each plant;

(c) hybridizing the matrices of step (b) with a cDNA clone selected from the cDNA clones of claim 7 marking a chromosome or unassigned linkage group which might contain DNA corresponding to said observable characteristic;

(d) comparing the hybridization pattern from each parent to determine the presence of polymorphisms;

(e) repeating steps (b) - (d) utilizing at least one additional cDNA clone selected from those marking other chromosomes which might contain DNA corresponding to the said observable characteristic and/or using additional restriction enzymes as necessary until polymorphisms have been identified by which the parents may be distinguished from each other by at least one difference on each chromosome or unassigned linkage group which might contain DNA corresponding to the said observable characteristic;

(f) crossing the parent plants to obtain an F1 generation and selfing or backcrossing the F1 generation to obtain a segregating generation containing individuals having the observable characteristic;

(g) selecting and scoring a statistically significant number of segregating individuals for the percent presence of the observable characteristic;

(h) extracting DNA from the segregating individuals of step (g) and following the procedures of steps (b) through (e) to obtain hybridized matrices for each individual showing polymorphisms when compared to the matrices of the parent not having the observable characteristic;

(i) identifying cDNA clones which are located within 10 map units (10 cM) of genes conferring the observable characteristic;

(j) if no such cDNA clones are identified, identifying cDNA clones which are located less than 30 map units (cM) from genes controlling the observable characteristic;

(k) selecting additional cDNA clones from the cDNA clones of claim 7, said cDNA clones having map positions spaced less than 30 map units (cM) from the cDNA clones of step (j), and hybridizing said clones with the segregating generation DNA of step (h);

(l) identifying cDNA clones which are located within 10 map units (10cM) from genes controlling the observable characteristic;

(m) if no such cDNA clones are identified, selecting additional cDNA clones from the cDNA clones of claim 7, said cDNA clones having map positions spaced closer to previously tested cDNA clones having high percent identity than those having a lower percent identify, hybridizing said clones with the DNA of segregating individuals of step (h) and identifying cDNA clones which are located less than 10 map units from genes controlling the observable characteristic.

(n) if no such cDNA clones are identified, repeating step (m) until such clones have been identified.

14. The method of claim 10 where isozyme markers are utilized in addition to cDNA clones and the presence or occurrence of each isozyme occurring in the parent having the observable characteristic is correlated with the percent presence of the observable characteristic in the segregating generation individuals.

15. The method of claim 10 including locating DNA controlling the observable characteristic on a chromosome map.

16. A method for screening a tomato plant for the presence of an observable characteristic comprising:

(a) linking the observable characteristic with a marker selected from the cDNA clones of claim 7; and

(b) testing the DNA of the tomato plant for the presence of RFLP's characteristic of the selected marker.

17. A method for transferring a desired characteristic from a tomato plant into a second tomato variety comprising:

(a) linking the desired characteristic with a previously mapped marker;

(b) probing DNA of the second tomato variety with cDNA of claim 4 to determine its DNA profile;

(c) crossbreeding a tomato plant having the desired characteristic with a plant of the second tomato variety to obtain an F1 generation;

(d) screening F1 individuals for being homozygous for the desired characteristic using the linked marker;

(e) probing the DNA of the F1 individuals having the desired characteristic with cDNA of claim 4 to determine their DNA profiles;

(f) selecting F1 individuals having the desired characteristic and otherwise having DNA profiles most similar to that of the parent of the second tomato variety for breeding;

(g) backcrossing the selected individuals of step (f) with a parent of the second tomato variety to produce an BC1 generation;

(h) screening BC1 individuals for the desired characteristic using the linked marker;

(i) probing the DNA of the BC1 individuals having the desired characteristic with cDNA of claim 4 to determine their DNA profiles;

(j) selecting BC1 individuals having the desired characteristic and otherwise having DNA profiles similar to that of the parent of the second tomato variety; and

(k) repeating steps (g) - (i) with BC2 and subsequent generations until individuals homozygous for the desired characteristic and having the desired degree of similarity to the parent of the second tomato variety are produced.

0 242 062

**1**
18 — Idh-1
Prx-1
21 — CD15
18 — Skdh-1
4 — CD24
17 — CD47
4 — CD12
27 — CD9B / CD52A
5 — CD9A / CD16B
5 — CD16A / CD20
10 — Act-3
1 — CD44 / CD28
18 — Est-3

**2**
1 — R45S / CD49B
7 — CD37
CD1
7 — Rbcs-1 3-91
15 — CD33A
9 — CD30B
14 — Cab-1; 3-131 / CD11
11 — Est-7
8 — Prx-2
6 — CD43
4 — CD35
6 — CD9C
13 — Rbcs-3
14 — CD30A / CD33B

**3**
12 — Cab-3
Pgm-1
18 — CD50
2 — Rbcs-2; 3-167
7 — CD13A / CD31B / CD6B / CD29C / Act-4 / CD52B / CD11
17 — CD51

**4**
11 — 6Pgdh-1
5 — CD59
11 — CD49A
15 — Act-1 / Tpi-2
4 — Pgm-2
9 — Adh-1
Act-10 / CD55

**5**
9 — CD41
8 — CD64
CD31A
10 — CD38B
6Pgdh-3

**6**
8 — CD14
7 — CD13B
11 — Aps-1
CD29B

**7**
3 — CD61 / CD58 / CD65
8 — CD48
16 — Got-3
7 — Aco-2
18 — Got-2
7 — Cab-4
9 — CD54 / CD57
6 — Hdh-2
18 — CD62

**8**
30 — CD29A
16 — CD46
10 — CD60
10 — CD7
10 — Cab-2; 3-185
12 — Aps-2
14 — CD40
1 — CD21A / CD16C

**9**

**10**
7 — CD56
10 — CD45
5 — Est-8
10 — CD38A
10 — CD34B
CD34A / Act-6

**11**

**12**
1 — CD21B / 6Pgdh-2 / CD27
7 — CD4B / CD16D
2 — CD6A
3 — Pgl-1
40 — Aco-1
10 — Cab-5; 3-48

Unassigned linkage groups:

CD32A — 11 — CD3 — 3 — CD8

Sod-1 / Act-5 — 7 — CD29D — 9 — CD25B

CD42 — 8 — CD25A

Act-2 — 6 — CD5 — 5 — CD32B

FIG. 1

FIG. 2A

FIG. 2B

FIG. 2C

0 242 062